Europäisches Patentamt

(19) European Patent Office

Office européen des brevets

(11) Publication number: **0 197 335 B1**

## (12) EUROPEAN PATENT SPECIFICATION

(45) Date of publication of patent specification:
06.02.91 Bulletin 91/06

(21) Application number: 86103171.4

(22) Date of filing: 10.03.86

(51) Int. Cl.⁵: **C12N 15/52**, C12N 15/77,
C07H 21/04, C12N 1/20,
// (C12R1/15, 1:13, 1:19,
1:07, 1:44, 1:425)

(54) Process for producing L-lysine.

The file contains technical information submitted after the application was filed and not included in this specification

(30) Priority: 12.03.85 JP 47515/85

(43) Date of publication of application:
15.10.86 Bulletin 86/42

(45) Publication of the grant of the patent:
06.02.91 Bulletin 91/06

(84) Designated Contracting States:
DE FR GB

(56) References cited:
WO-A-84/03301
FR-A- 2 482 622

(73) Proprietor: KYOWA HAKKO KOGYO CO., LTD.
6-1, Ohte-Machi Itchome
Chiyoda-ku Tokyo (JP)

(72) Inventor: Katsumata, Ryoichi
Popuraoka Coopu 6 401 2-12-3, Naruse
Machida-shi Tokyo (JP)
Inventor: Mizukami, Toru
1-6-16, Asahi-machi
Machida-shi Tokyo (JP)
Inventor: Oka, Tetsuo
2360-17, Nara-machi
Midori-ku Yokohama-shi Kanagawa-ken (JP)

(74) Representative: Vossius & Partner
Siebertstrasse 4 P.O. Box 86 07 67
D-8000 München 86 (DE)

Jouve, 18, rue Saint-Denis, 75001 PARIS

## Description

The present invention relates to the field of biotechnology and particularly to the application of recombinant DNA technology to microorganisms involved in the production of L-lysine. More particularly, the invention relates to the molecular cloning of a gene encoding an enzyme involved in the biosynthesis of L-lysine and the expression of said gene in Corynebacteria or Brevibacteria.

Improvement of the so-called glutamic acid-producing microorganisms such as microorganisms of the genera Corynebacterium and Brevibacterium by recombinant DNA technology to increase their L-lysine productivity is disclosed in the Japanese Published Unexamined Patent Applications Nos. 160997/81 and 126789/83.

Large-scale production of L-lysine, however, still remains difficult and it is desirable to improve the L-lysine productivity of the above bacteria by a more efficient utilization of recombinant DNA technology. Thus, in employing recombinant DNA technology, the technical problem underlying the invention is to provide

1. a DNA fragment comprising a gene coding for dihydrodipicolinic acid synthase (DDPS)

2. a process for producing L-lysine by expressing said gene in Corynebacteria or Brevibacteria

3. bacterial transformants carrying a recombinant DNA comprising said DNA fragment.

The solution of the problem is evident from claims 1 to 4.

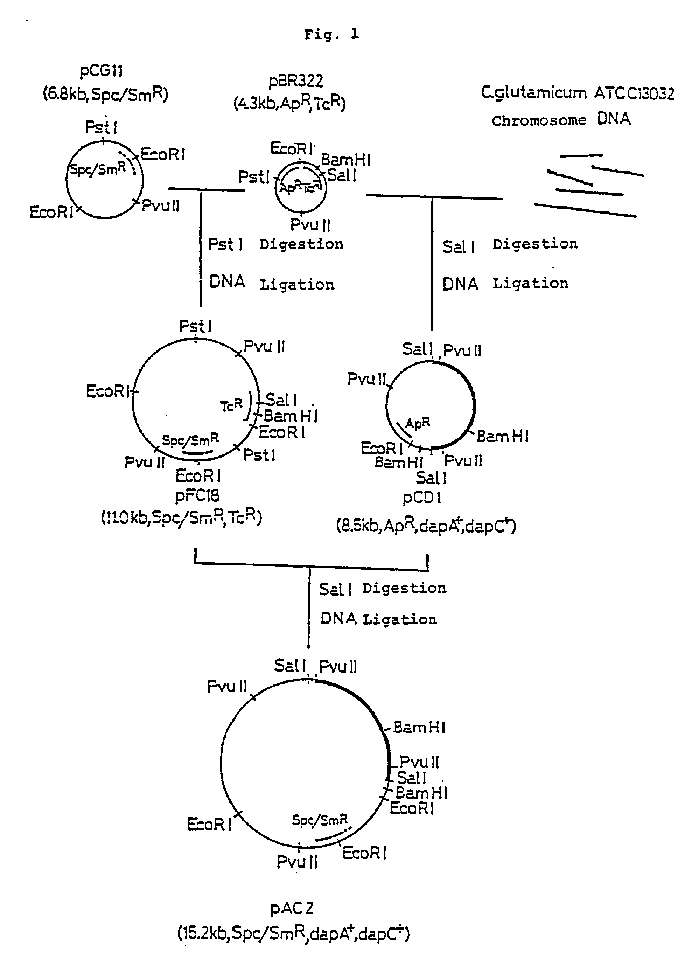

Fig. 1 is a flow sheet showing a process for preparing vector plasmid pFC18, wherein Spc/Sm represents the resistance markers spectinomycin and streptomycin, Ap represents ampicillin, and Tc tetracycline.

Any of the microorganisms known as the glutamic acid-producing microorganisms belonging to the genus Corynebacterium or Brevibacterium can be used as a host for the recombinant vector. Preferably, the following strains can be used.

| | |
|---|---|
| Corynebacterium glutamicum | ATCC 13032 |
| Corynebacterium acetoacidophilum | ATCC 13870 |
| Corynebacterium herculis | ATCC 13868 |
| Corynebacterium lilium | ATCC 15990 |
| Brevibacterium divaricatum | ATCC 14020 |
| Brevibacterium flavum | ATCC 14067 |
| Brevibacterium immariophilum | ATCC 14068 |
| Brevibacterium lactofermentum | ATCC 13869 |
| Brevibacterium thiogenitalis | ATCC 19240 |

Wild-type strains without lysine-productivity can be used as hosts, but strains having lysine-productibity can also be used. Lysine-producing strains, such as e.g. amino acid-requiring mutant strains, amino acid analog-resistant mutant strains, etc. are applicable.

The gene involved in the synthesis of DDPS, can be derived from eukaryotes, prokaryotes, viruses, bacteriophages or plasmids. The gene of a bacterial strain for example, of the genus Escherichia, Corynebacterium, Brevibacterium, Microbacterium, Bacillus, Staphylococcus, Streptococcus, or Serratia, is preferable. The gene derived from a lysine-producing mutant strain belonging to these bacteria is particularly preferable.

As vectors for incorporating the DNA fragment containing the gene, pCG1, pCG2, pCG4, pCG11, pCE54, pCB101, etc. are preferably used. Processes for producing these vectors are described in Japanese Published Unexamined Patent Application Nos. 134500/82, 183799/82, 35197/83 and 105999/83.

The recombinant DNA comprising the DNA fragment containing the gene involved in the synthesis of DDPS and the vector DNA can be obtained by standard recombinant DNA techniques which comprises cleaving in vitro both DNAs with restriction enzymes, ligating the cleaved DNAs with DNA ligase, transforming a DDPS deficient mutant strain belonging to the genus Corynebacterium or Brevibacterium with the ligation mixture, and selecting transformants with restored DDPS activity. The recombinant DNA technology can be carried out according to the Japanese Published Unexamined Patent Applications Nos 186492/82 and 186489/82.

Instead of cloning the recombinant DNA directly in Corynebacteria or Brevibacteria, the recombinant DNA can also be obtained by using another well-established host-vector system as exemplified by the

2

Escherichia coli system. That is, cloned DNA fragments containing the gene can be obtained from Escherichia coli transformants. This method comprises transforming an Escherichia coli mutant lacking the gene involved in the synthesis of DDPS with the in vitro ligation mixture of the donor DNA and the vector DNA, and selecting transformants with a restored phenotype. The cloned DNA fragment can be recovered from the Escherichia coli plasmid and combined in vitro with a vector for Corynebacteria and Brevibacteria. The subsequent transformation of the above bacteria leads to the desired bacterial strain belonging to the genus Corynebacterium or Brevibacterium harbouring the desired gene.

The present invention is explained in more detail below, with reference to a gene involved in the synthesis of DDPS of Corynebacterium glutamicum (hereinafter referred to as DDPS gene or dapA).

A DNA fragment containing dapA of Corynebacterium glutamicum can be cloned in advance using the host-vector system of Escherichia coli. According to the procedure as described, for example, in Methods in Enzymology, Volume 68, edited by Ray Wu and published by Academic Press, New York (1979), the following steps are performed :

Chromosomal DNA from Corynebacterium glutamicum ATCC 13032, and the Escherichia coli, vector plasmid pBR322 (having a resistance to ampicillin and tetracycline) are cleaved by the restriction enzyme SalI, and then ligated by T4-DNA ligase.

Substrain TM103 of Escherichia coli K12 [hsdR⁻(host-specific restriction defective) and dapA⁻ (DDPS-defective : diaminopimelic acid-requiring)] is transformed by the ligated product, and the transformants capable of growing on a minimal medium containing ampicillin are selected. The plasmid is recovered by a conventional method from a transformant not requiring diaminopimelic acid and resistant to ampicillin. Further, the plasmid DNA is cleaved with restriction enzymes and the DNA fragments thus obtained are analyzed by agarose gel electrophoresis, whereby the structure of the plasmid can be determined. One of the thus obtained plasmids is pCD1. pCD1 carries a SalI DNA fragment of 4.2 kilobases (Kb) in the unique SalI cleavage site of pBR322 (Fig. 1).

It was proved by complementation studies that the plasmid pCD1 contains the DDPS gene : all ampicillin resistant transformants of the DDPS deficient E. coli mutant AT998 (Hfr, dapA16 ; see J. Bacteriol. 105 (1971), 844), transformed with said plasmid are rendered diaminopimelic acid (DAP) independent. Another Escherichia coli mutant AT997 (Hfr, dapC15 ; see J. Bacteriol. 105, (1971), 844) which also requires DAP for growth is also rendered DAP independent by transformation with the plasmid pCD1. The 4.2 Kb SalI fragment of the plasmid pCD1 was recloned in the shuttle vector pFC18 which is capable of replicating in bacteria of the genera Corynebacterium, Brevibacterium and Escherichia. The shuttle vector pFC18 (having a resistance to tetracycline and a resistance to spectinomycin) was prepared by inserting the DNA of the Corynebacteria and Brevibacteria plasmid pCG11 (see Japanese Published Unexamined Patent Application No. 134500/82) into the PstI-site of the E. coli plasmid pBR 322. The insertion of the 4.2 Kb SalI-fragment into pFC18 results in the plasmid pAC2. The process for preparing said plasmid and its structure are shown in Fig. 1.

pAC2 is prepared by the following steps :

pFC18 and pCD1 plasmid DNAs are cleaved with the restriction enzyme SalI, then mixed together and subjected to the action of T4-DNA ligase. Then the DDPS-defective substrain TM103 of Escherichia coli K12 is transformed with the ligation mixture, and the transformants growing on a minimal medium containing spectinomycin are selected. Plasmid DNA is extracted from the culture of one of the thus obtained transformants having resistance to spectinomycin and not requiring diaminopimelic acid.

The SalI cleavage products of the plasmid DNA are separated by agarose gel electrophoresis to investigate the structure of the plasmid. It is confirmed that the SalI DNA fragment of 4.2 Kb derived from pCD1 is inserted in the SalI cleavage site of pFC18. Then, the DDPS-defective mutant strain AT998 and the mutant strain AT997 which are substrains of Escherichia coli K12 strain are transformed with said plasmid, and because the defect in the mutants is restored, it is confirmed that at least dapA of Corynebacterium glutamicum cloned in pCD1 is incorporated in said plasmid. Said plasmid is named pAC2 (Fig. 1).

Corynebacterium glutamicum RH6 strain, a lysine-producing microorganism having a homoserine requirement caused by mutation (the strain is deposited as FERM-BP 704 in Fermentation Research Institute, Agency of Industrial Science and Technology) is transformed with the plasmid pAC2. The transformation can be carried out according to a method using protoplast of strains of the genus Corynebacterium or Brevibacterium previously found by the present inventors, (Japanese Published Unexamined Patent Application Nos. 186492/82 and 186489/82). Protoplasts of Corynebacterium glutamicum strain RH6 are transformed according to said method, and then transformants are selected on a regeneration medium containing spectinomycin. The plasmid separated from the culture of one of the thus obtained spectinomycin-resistant transformants is subjected to the same restriction enzyme cleavage and agarose gel electrophoresis as described in the preceding paragraph, and also to a test for the confirmation of the presence of dapA by transforming of Escherichia coli K12 substrains AT998 and AT997. It is confirmed that pAC2 plasmid is introduced into RH6 strain.

The production of L-lysine by pAC2 transformants can be carried out according to a culturing method used in the conventional process for producing L-lysine by fermentation. That is, when the transformant is cultured in a conventional medium containing a carbon source, a nitrogen source, inorganic matters, amino acids, vitamins, etc. under aerobic conditions, while controlling the temperature, pH, etc., lysine is formed and accumulates in the culture broth. Thus, L-lysine can be recovered from the culture broth according to known methods for example, by active carbon treatment, ion exchange resin treatment, etc.

Thus, L-lysine can be produced in a higher yield by using a pAC2-containing strain of the genus Corynebacterium or Brevivacterium than by using a strain which does not contain pAC2. The usefulness of the present invention lies in an endowment or an enhancement of L-lysine productivity by introducing a recombinant DNA in which the DDPS gene and a vector of a strain of the genus Corynebacterium or Brevibacterium are combined in a form capable of expressing the gene in a strain of the genus Corynebacterium or Brevibacterium. In the present specification, an example of using the gene involved in the synthesis of DDPS derived from Corynebacterium glutamicum is shown. Lysine productivity of lysine-producing microorganisms can also be improved when the corresponding genes of other organisms are used in place of said gene.

The plasmid vector only provides for stable inheritance of the gene involved in the synthesis of DDPS. Thus, not only pFC18 exemplified in the present specification, but also other plasmids autonomously replicable in a microorganism of the genus Corynebacterium or Brevibacterium and phage vectors capable of stable inheritance by being inserted in the chromosome of the host can be used.

In spite of many common microbiological properties, microorganisms with high glutamic acid productivity (so-called glutamic acid-producing microorganisms) are classified as comprising various species and even genera such as Corynebacterium or Brevibacterium probably because of their industrial importance. However, it has been pointed out that these microorganisms should be classified as one species because they shave a large degree of homology in the amino acid sequences of their cell wall proteins and in the GC content of their DNAs. Recently, it has been reported that these microorganisms have more than 70%-80% homology in their DNA sequences as shown by DNA-DNA hybridization studies, indicating that the microorganisms are very closely related [Komatsu, Y. : Report of the Fermentative Research institute, No. 55,1 (1980), and Suzuki, K., Kaneko, T., and Komagata, K. : Int. J. Syst. Bacteriol., 31, 131 (1981)].

In the present specification, a case where a recombinant DNA is introduced into Corynebacterium glutamicum RH6, and where the improvement in L-lysine production depends on the expression of the gene is presented. Considering the above mentioned very close relationship of glutamic acid-producing microorganisms, it is readily assumed that the present invention is applicable to all the glutamic acid-producing microorganisms. The effect of the present invention depends on whether the recombinant DNA autonomously replicates in the glutamic acid-producing microorganism and whether the gene is expressed, and therefore slight differences in the DNA sequences between glutamic acid-producing microorganisms are unimportant. That the glutamic acid-producing microorganisms shave the ability to allow replication of plasmids and expression of genes is apparent from the fact that plasmid pCG4 which is isolated from Corynebacterium glutamicum 225-250 (Japanese Published Unexamined Patent Application No. 183799/82) and which has spectinomycin and/or streptomycin resistance gene(s) can be generally replicated and expressed in glutamic acid-producing microorganisms such as strains of the genera Corynebacterium and Brevibacterium (Jananese Published Unexamined Patent Application NO. 1864492/82). Futher, it has been shown by the present inventors that the genes involved in the biosynthesis tryptophane and histidine of a Brevibacterium can be expressed in a bacterium of the genus Cirynebacterium (Japanese Published unexamined Patent Application Nos. 25398/83 and 25397/83). Thus, it is apparent that the genes can be mutually expressed between the bacteria of thes two genera. Accordingly, the bacteria to which the present invention is applicable include not only Corynebacterium glutamicum but also all the glutamic acid-producing microorganisms including the bacteria of the genera Corynebacterium and Brevibacterium.

Certain specific embodiments of the present invention are illustrated by the following representative examples.


Example 1


(1) Construction of a substrain TM103 of Escherichia coli K12 having a host-specific restriction-deficient mutation and a DDPS-deficient mutation :

To more readily clone a gene involved in the synthesis of DDPS of Corynebacterium glutamicum, which is a foreign gene, in a host-vector system of Escherichia coli, a strain of Escherichia coli having the host-specific restriction-deficient mutation (hsdR2⁻) and the DDPS-deficient mutation (dapA 16⁻) simultaneously was constructed as a host microorganism in the following manner.

From a substrain WA802 of Escherichia coli K12 having a restriction-deficient mutation (Escherichia coli K12 WA802, FERM BP-718) [F⁻ met Bl hsd R2 : J. Mol. Biol. 16 118 (1966)] was derived a spontaneous mutant strain RF82 having a resistance to 25 μg/mℓ of rifampicin. The strain RF82 and a DDPS-defective mutant strain AT998 (Hfr dapA16) (Escherichia coli K12 AT998, FERM BP-720) were cultured in L medium [1% bactotrypton (Difco, 0.5% yeast extract (Daigo Eiyo Kagaku K.K., Japan), and 0.5% NaCl, adjusted to pH 7.0 with NaOH) containing 50 μg/mℓ of diaminopimelic acid at 37°C for 3 hours. After washing twice with a physiological saline solution by centrifugation, the washed cells were spread on M9 minimal medium agar plates (a medium containing 2 g of glucose, 1 g of NH₄Cl, 6 g of Na₂HPO₄, 3 g of KH₂PO₄, 0.1 g of MgSO₄·7H₂O, 15 mg of CaCl₂·2H₂O, 4 mg of thiamine hydrochloride and 15 g of agar in 1 ℓ of water and adjusted to pH 7.2) containing 25 μg/mℓ of rifampicin and 50 μg/mℓ of diaminopimelic acid, and transconjugants having resistance to rifampicin and not requiring methionine were selected. Strains showing diaminopimelic acid requirement and having a restriction-deficient mutation were selected from the transconjugants, and one of those strains was designated as TM103. The presence of the restriction-deficient mutation was determined by the plating efficiency of λ phage propagated on the strain C600r⁻m⁻ (ATCC 33525), a modification-deficient Escherichia coli K12 strain [M. Melselson : J. Mol. Biol., 9 734 (1964)]. λ Phage was obtained from Escherichia coli K12 λ lysogenic bacterium ATCC 10798 according to a conventional method. A strain showing the same plating efficiency as that of WA802 was regarded as a transconjugant having a restriction-deficient mutation.

(2) Cloning of DDPS gene (dapA) of Corynebacterium glutamicum :

Cloning was carried out by using a host-vector system of Escherichia coli. pBR322 used as a vector was a commercially available product made by Takara Shuzo Co., Japan. Chromosomal DNA used as a donor DNA was isolated from Corynebacterium glutamicum ATCC 13032 according to a procedure previously disclosed by the present inventors [Japanese Published Unexamined Patent Application No. 126789/83, Example 1, Item (1)].

12 units of the restriction enzyme salI (product of Takara Shuzo Co., Japan) were added to 120 μℓ of a reaction solution for the restriction enzyme SalI (10 mM Tris-HCl, 7 mM MgCl₂, 100mM NaCl, pH 7.5) containing 4 μg of pBR322 DNA and 8 μg of chromosomal DNA of Corynebacterium glutamicum. The mixture was incubated at 37°C for 60 minutes, and the reaction was stopped by heating at 65°C for 10 minutes 30 μℓ of T₄-DNA ligase buffer solution (660 mM Tris-HCl, 66 mM MgCl₂, 100 mM dithiothreitol, pH 7.6), 30 μℓ of 5 mM ATP, 0.3 units of T₄-DNA ligase (product of Takara Shuzo Co., Japan), and 120 μℓ of H₂O were added to the digested product. The mixture was incubated at 12°C for 16 hours.

The ligation reaction mixture was used for the transformation of Escherichia coli k12 substrain TM103. Competent cells of TM103 were prepared according to the method of Dagert et al [Dagert, M. et al : Gene 6, 23 (1979)]. That is, strain TM103 was inoculated in 50 mℓ of L medium supplemented with 50 μg/mℓ of diaminopimelic acid and cultured at 37°C until the absorbance (OD) at 660 nm reached 0.5 as measured by Tokyo Koden colorimeter. The culture was cooled in ice water for 10 minutes, and then the cells were collected by centrifugation and suspended in 20 mℓ of cooled 0.1 M calcium chloride. The suspension was kept at 0°C for 20 minutes. The cells were collected by centrifugation and resuspended in 0.5 mℓ of 0.1 M calcium chloride. The suspension was left standing at 0°C for 18 hours. To 400 μℓ of the suspension were added 200 μℓ of the ligation reaction mixture prepared above, and the mixture was kept 0°C for 10 minutes and then heated at 37°C for 5 minutes. Then, 9 mℓ of L medium containing 50 μg/mℓ diaminopimelic acid were added thereto, and the mixtures was incubated under shaking at 37°C for 2 hours. The cultured cells were washed twice with a physiological saline solution following collection of the cells by centrifugation, and spread on M9 minimal medium agar plates containing 50 μg/mℓ ampicillin, and cultured at 37°C for 4 days. The thus obtained transformants having resistance to ampicillin and not requiring diaminopimelic acid were subjected to single colony isolation on L medium agar plates (L medium containing 1.5% agar) containing 50 μg/mℓ ampicillin.

Plasmid DNA was isolated from the cultured cells of purified transformants according to the method of An et al [An, G. et al : J. Bacteriol., 140 400 (1979)]. The plasmid DNA was digested with restriction enzymes and the fragments had analyzed by agarose gel electrophoresis. It was found that the plasmid DNA had such a structure that the SalI DNA fragment of 4.2 Kb was inserted in the unique SalI cleavage site of pBR322. The plasmid was named pCD1.

pCD1 was used to transform the DDPS-defective mutant strain AT998 and THPS-the mutant strain AT997, which were substrains of Escherichia coli k12. Transformation of strain AT998 and strain AT997 (Escherichia coli K12 AT997, FERM BP-719) was carried out in the same manner as the transformation of strain TM103. None of the transformants having a resistance to ampicillin obtained from these two strains required diaminopimelic acid. It is evident from these facts that the DDPS gene (dapA) of Corynebacterium glutamicum exists on the 4.2 Kb Sall DNA fragment cloned in pCD1.

The restriction-deficient mutation of the strain TM103 which was used in the cloning step was utilized merely to increase the yield of transformants cloning, and strains without such a mutation can also be used as host microorganisms.

(3) Preparation of plasmid pAC2 :

The 4.2 Kb Sall DNA fragment containing the dapA gene derived from Corynebacterium glutamicum was recloned in the shuttle plasmid vector resistance to spectinomycin and a resistance to tetracycline). Preparation of pFC18 was carried out according to the following procedure.

pCG11 was isolated from a strain containing pCG11 (ATCC 39022) according to the procedure previously disclosed by the present inventors [Japanese Published Unexamined Patent application No. 134500/82, Example 1, Item (1)]. pBR322 user for this purpose was a commercially available product of Takara Shuzo Co., Japan.

8 units of the restriction enzyme PstI (product of Takara Shuzo Co., Japan) were added, to 120 $\mu\ell$ of a reaction solution (20 mM Tris-HCl (pH 7.5), 10 mM $MgCl_2$, 50 mM $(NH_4)_2SO_4$ and 0.01% bovine serum albumin) containing 4 $\mu$g each of pCG11 and pBR322 plasmid DNAs. The mixture was incubated at 37°C for 60 minutes. The reaction was stopped by heating at 65°C for 10 minutes. Then, 30 $\mu\ell$ of $T_4$-DNA ligase buffer solution, 30 $\mu\ell$ of 5 mM ATP, 0.3 units of $T_4$-DNA ligase and 120 $\mu\ell$ of $H_2O$ were added thereto, and the mixture was incubated at 12°C for 16 hours. Substrain WA802 of Escherichia coli K12 was transformed with the ligation reaction product according to the procedure described in Example 1, Item (2). One of the transformants propagated on L medium agar plates containing 100 $\mu$g/m$\ell$ spectinomycin and 25 $\mu$g/m$\ell$ tetracycline was subjected to single colony isolation on the same agar medium, and plasmid DNA was isolated isolated from the cultured cells of the purified strain according to the method of An et al. The structure of the plasmid DNA was investigated by restriction enzyme cleavage and agarose gel electrophoresis of the obtained fragment and it was found that the plasmid DNA had such a structure that pBR322 was inserted in the unique PstI cleavage site of pCG11 (Fig. 1). The plasmid was named pFC18.

A recombinant plasmid containing dapA derived from Corynebacterium glutamicum was prepared, using pFC18 as a vector according to the following procedure. 8 units of the restriction enzyme Sall were added, to 120 $\mu\ell$ of a reaction solution for Sall containing 4 $\mu$g each of pFC18 and pCD1 plasmid DNAs and the mixture was incubated at 37°C for 60 minutes. The reaction was stopped by heating at 65°C for 10 minutes, and then 30 $\mu\ell$ of $T_4$-DNA ligase buffer solution, 30 $\mu\ell$ of 5 mM ATP, 0.3 units of $T_4$-DNA ligase and 120 $\mu\ell$ of $H_2O$ were added thereto. The mixture was incubated at 12°C for 16 hours.

The ligation reaction product was used to transform the Escherichia coli strain TM103. Transformation was carried out according to the procedure described in Example 1, Item (2), and transformants were selected on M9 minimal medium agar plates containing 100 $\mu$g/m$\ell$ of spectinomycin. From one of the thus obtained transformants having a resistance to spectinomycin and not requiring diaminopimelic acid was isolated plasmid DNA according to the method of An et al. The structure of the plasmid was investigated by restriction enzyme cleavage and agarose gel electrophoresis. It was found that the plasmid had such a structure that the 4.2 Kb Sall DNA fragment derived from pCD1 was inserted in the single Sall cleavage site of pFC18. The plasmid was named pAC2.

Transformation with pAC2 of said AT998 and AT997 confirmed that the spectinomycin-resistant transformants were rendemed to be diaminopimelic acid independent at the same time.

(4) Introduction of pAC2 into Corynebacterium glutamicum strain RH6 :

Transformation of Corynebacterium glutamicum strain RH6 having a homoserine requirement) was carried out using pAC2.

0.1 m$\ell$ of a starting culture of strain RH6 was inoculated in 10 m$\ell$ of SSM medium (a medium containing 10 g of glucose, 4 g of $NH_4Cl$, 2 g of urea 1 g of yeast extract, 1 g of $KH_2PO_4$, 3 g of $K_2HPO_4$, 0.4 g of $MgCl_2 \cdot 6H_2O$, 10 mg of $FeSO_4 \cdot 7H_2O$, 0.2 mg of $MmSO_4 \cdot 4\text{-}6H_2O$, 0.9 mg of $ZnSO_4 \cdot 7H_2O$, 0.4 mg of $CuSO_4 \cdot 5H_2O$, 0.09 mg of $Na_2B_4O_7 \cdot 10H_2O$, 0.04 mg of $(NH_4)_6 Mo_7O_{24} \cdot 4H_2O$, 30 $\mu$g of biotin, and 1 mg of thiamine hydrochloride in 1 $\ell$ of water and adjusted to pH 7.2) containing 50 $\mu$g/m$\ell$ homoserine, and cultured under shaking at 30°C. When the OD at 660 nm reached 0.15, penicillin G was added thereto to a final concentration of 0.5 units/m$\ell$. Culturing was further continued, and when OD reached about 0.6, the cultured cells were collected, and suspended in 2 m$\ell$ of RCGP medium [a medium containing 5 g of glucose, 5 g of casamino acid, 2.5 g of yeast extract, 3.5 g of $K_2HPO_4$, 1.5 g of $KH_2PO_4$, 0.41 g of $MgCl_2 \cdot 6H_2O$, 10 mg of $FeSO_4 \cdot 7H_2O$, 2 mg of $MmSO_4 \cdot 4\text{-}6$ $H_2O$, 0.9 mg of $ZnSO_4 \cdot 7H_2O$, 0.04 mg of $(NH_4)_6MO_7O_{24} \cdot 4H_2O$, 30 $\mu$g of biotin, 2 mg of thiamine hydrochloride, 135 g of disodium succinate, and 30 g of polyvinyl-pyrrolidone (molecular weight : 10,000) in 1 $\ell$ of water and adjusted to pH 7.2] containing 1 mg/m$\ell$ lysozyme and the suspension was shaker gently at 30°C for 14 hours to make protoplasts.

Then, 1 mℓ of the protoplast solution was centrifuged at 2,500 x g for 15 minutes to precipitate the protoplasts. The protoplasts were suspended in 1 mℓ of TSMC buffer solution (containing 10 mM MgC1$_2$, 30 mM CaCl$_2$, 50 mM Tris-HCl of pH 7.5 and 400 mM sucrose) and washed following centrifugation. The protoplasts were resuspended in 0.1 mℓ of TSMC buffer solution. To the suspension 20 μg of the plasmid pAC2 were added, followed by the addition of 0.8 mℓ of TSMC buffer solution containing 20% (w/v) polyethyleneglycol (PEG) 6,000. After 3 minutes, 2 mℓ of RCGP medium was added to the mixture, and the protoplasts were precipitated by centrifugation at 2,500 x g for 5 minutes. The protoplasts were suspended in 1 mℓ of RCGP, and culturing was carried out with gentle shaking at 30°C for 2 hours. 0.1 mℓ of the protoplast suspension was spread on RCGP agar medium (RCGP madium containing 1.4% agar) containing 400 μg/mℓ spectinomycin, and cultured at 30°C for 6 days. Plasmid DNA was extracted from one of the thus obtained spectinomycin-resistant transformants according to the procedure previously disclosed by the present inventors [Japanes Published Unexamined Patent Application No. 134500/82, Example 1, Item (1)]. Restriction enzyme cleavage and subsequent agarose gel electrophoresis of the thus obtained fragments had all shown that the isolated plasmid had the same structure as pAC2. It was also shown by a transformation test analog out to that p$^+$ the foregoing item that the plasmid restores the diaminopimelic acid independence of the strains AT998 and AT997 as pAC2 does. The strain which was thus confirmed to be transformed by pAC2 was named Corynebacterium glutamicum RH6/pAC2.

(5) Production of L-lysine by pAC2-containing strain :

Production of L-lysine by Corynebacterium glutamicum strain RH6/pAC2 and parent strain RH6 which do not contain the plasmid was carried out in the following manner.

Strain RH6/pAC2 and strain RH6 were separately cultured with shaking in 3 mℓ of NB medium (a medium containing 20 g of bouillon and 5 g of yeast extract in 1 ℓ of water and adjusted to pH 7.2) at 30°C for 16 hours. Then, 0.5 mℓ of the cultured broth was inoculated in 5 mℓ of a production medium L1 [a medium containing 100 g of glucose, 30 g of (NH$_4$)$_2$SO$_4$, 0.5 g of KH$_2$PO$_4$, K$_2$HPO$_4$, 1 g of MgSO$_4$·7H$_2$O, 10 mg of FeSO$_4$·7H$_2$O, 10 mg of MmSO$_4$·4-6 H$_2$O, 100 μg of boitin, 200 mg of homoserine, and 30 g of calcium carbonate in 1 ℓ of water and adjusted to pH 7.2], and cultured with shaking at 30°C for 72 hours. After culturing, the L-lysine produced in the filtrate of culture liquid was quantitatively determined by colorimetry according to the acidic copper ninhydrin procedure [Chinard, F.D. : J. Biol. Chem. 199, 91 (1952)]. The results are shown in Table 1. It is apparent from the Table that the recombinant plasmid pAC2 containing dapA of Corynebacterium glutamicum can enhance the L-lysine productivity of the strain RH6.

## Table 1

Production of L-lysine by Corynebacterium glutamicum RH6 strain and plasmid pAC2-introduced strain

| Strain | L-lysine (mc/mℓ) |
|---|---|
| Corynebacterium glutamicum RH6 | 14.8 |
| Corynebacterium glutamicum RH6/pAC2 | 20.0 |

## Claims

1. A DNA fragment comprising a gene coding for dihydrodipicolinic acid synthase isolated from Corynebacterium glutamicum, said fragment having cleavage sites for Sall at both ends, one cleavage site for BamHI and two cleavage sites for Pvull and a size of about 4.2 kilobases.

2. A process for producing L-lysine which comprises culturing in a medium a microorganism belonging to the genus Corynebacterium or Brevibacterium carrying a recombinant DNA comprising the DNA fragment, according to claim 1, accumulating L-lysine in the medium, an recovering L-lysine therefrom.

3. The process according to Claim 2, wherein the microorganism is selected from Corynebacterium glutamicum, Corynebacterium herculis, Corynebacterium lilium, Corynebacterium acetoacidophilum, Brevibacterium flavum, Brevibacterium lactofermentum, Brevibacterium divaricatum, Brevibacterium immariophilium, or Brevibacterium thiogenitalis.

4. A microorganism belonging to the genus Corynebacterium or Brevibacterium, which carries a recombinant DNA comprising the DNA fragment defined in claim 1.

## Ansprüche

1. DNA-Fragment umfassend ein aus Corynebacterium glutamicum isoliertes Dihydrodipicolinsäure-Synthase codierendes Gen, wobei das Fragment an beiden Enden Spaltstellen für Sall, eine Spaltstelle für BamHI und zwei Spaltstellen für Pvull und eine Größe von etwa 4,2 Kilobasen aufweist.

2. Verfahren zur Herstellung von L-Lysin, dadurch gekennzeichnet, daß man in einem Medium ein zur Gattung Corynebacterium oder Brevibacterium gehörenden Mikroorganismus züchtet, der eine rekombinante, das DNA-Fragment nach Anspruch 1 umfassende DNA trägt, das L-Lysin in dem Medium anreichert und das L-Lysin daraus gewinnt.

3. Verfahren nach Anspruch 2, wobei der Mikroorganismus ausgewählt wurde aus Corynebacterium glutamicum, Corynebacterium herculis, Corynebacterium lilium, Corynebacterium acetoacidophilum, Brevibacterium flavum, Brevibacterium lactofermentum, Brevibacterium divaricatum, Brevibacterium immariophilium oder Brevibacterium thiogenitalis.

4. Mikroorganismus zur Gattung Corynebacterium oder Brevibacterium gehörend, der eine rekombinante, das in Anspruch 1 definierte DNA-Fragment umfassende DNA trägt.

## Revendications

1. Fragment d'ADN comprenant un gène codant pour l'acide dihydrodipicolinique synthétase, isolé de Corynebacterium glutamicum, ledit fragment comportant des sites de coupure par sall aux deux extrémités, un site de coupure par BamHI et deux sites de coupure par Pvull, et ayant une taille d'environ 4,2 kilobases.

2. Procédé de production de la L-lysine, qui comprend la culture, dans un milieu, d'un microorganisme appartenant au genre Corynebacterium ou Brevibacterium, portant un ADN recombinant comprenant le fragment d'ADN conforme à la revendication 1, l'accumulation de la L-lysine dans le milieu et la récupération de la L-lysine à partir de celui-ci.

3. Procédé conforme à la revendication 2, dans lequel le microorganisme est choisi parmi Corynebacterium glutamicum, Corynebacterium herculis, Corynebacterium lilium, Corynebacterium acetoacidophilum, Brevibacterium flavum, Brevibacterium lactofermentum, Brevibacterium divaricatum, Brevibacterium immariophilium, et Brevibacterium thiogenitalis.

4. Microorganisme appartenant au genre Corynebacterium ou Brevibacterium, qui porte un ADN recombinant comprenant le fragment d'ADN défini dans la revendication 1.

Fig. 1